# EUROPEAN PATENT APPLICATION

(11) **EP 4 571 650 A1**
(43) Date of publication of application: **18.06.2025**
(21) Application number: 23216049.9
(22) Date of filing: 12.12.2023
(51) Int. Cl.: G06T 11/00, G06N 3/0475, G06N 3/08, G06T 5/60, G06V 10/82, G16H 30/40

(54) **GENERATING SYNTHETIC IMAGES**

(71) Applicant: Bayer AG, 51373 Leverkusen (DE)
(72) Inventor: HOOGE, Jens, 13086 Berlin (DE)
(74) Representative: BIP Patents

(57) **Abstract**

Systems, methods, and computer programs disclosed herein relate to training a machine learning model and using the trained machine learning model to generate synthetic images, preferably synthetic medical images.

## Description

### FIELD OF THE DISCLOSURE

Systems, methods, and computer programs disclosed herein relate to training a machine learning model and using the trained machine learning model to generate synthetic images, preferably synthetic medical images.

### BACKGROUND

Artificial intelligence is increasingly finding its way into medicine. Machine learning models are being used not only to recognize signs of disease in medical images of the human or animal body (see, for example, WO2018202541A1, WO2020229152A1), but also increasingly to generate synthetic (artificial) medical images.

For example, WO2019/074938A1 and WO2022184297A1 describe methods for generating an artificial radiological image showing an examination region of an examination object after application of a standard amount of a contrast agent, although only a smaller amount of contrast agent than the standard amount was applied. The standard amount is the amount recommended by the manufacturer and/or distributor of the contrast agent and/or the amount approved by a regulatory authority and/or the amount listed in a package insert for the contrast agent. The methods described in WO2019/074938A1 and WO2022184297A can therefore be used to reduce the amount of contrast agent.

For example, WO2021052896A1 and WO2021069338A1 describe methods for generating an artificial medical image showing an examination region of an examination object in a first time period. The artificial medical image is generated using a trained machine learning model based on medical images showing the examination region in a second time period. The method can be used, for example, to speed up radiological examinations. Instead of measuring radiological images over a longer period of time, radiological images are measured only within a part of the time period and one or more radiological images are predicted for the remaining part of the time period using the trained model.

For example, US11170543B2B2 and US11181598B2 describe methods for MRI image reconstruction from undersampled data using machine learning models.

For example, WO2016/175755A1 and WO2014/036473A1 describe methods for generating a high radiation dose CT image based on a low radiation dose CT image using machine learning models.

Synthetic medical images, which are generated with the help of trained machine learning models, are often blurred, i.e., structures in the synthetic medical images are often less recognizable than in the measured medical images on which the synthetic medical images are based.

### SUMMARY

The present disclosure addresses this problem. The independent patent claims disclose means for generating improved synthetic images. Preferred embodiments of the present disclosure are found in the dependent patent claims, in the present description and in the drawings.

Thus, in a first aspect, the present disclosure relates to a computer-implemented method of training a machine learning model to generate synthetic images, the method comprising:
- providing a machine learning model, wherein the machine learning model is configured to generate a synthetic image based on input data and parameters of the machine learning model,
- providing training data, wherein the training data comprises, for each examination object of a plurality of examination objects, (i) input data and (ii) target data, wherein the target data comprises a target image of an examination region of the examination object,
- for each target image: determining target image features based on the target image,
- training the machine learning model, wherein the training comprises, for each examination object of the plurality of examination objects:
   ∘ inputting the input data into the machine learning model,
   ∘ receiving a synthetic image as an output of the machine learning model,
   ∘ determining image features based on the synthetic image,
   ∘ reducing deviations (i) between the synthetic image and the target image and (ii) between the image features of the synthetic image and the target image features by modifying model parameters,
- outputting and/or storing the trained machine learning model and/or transferring the trained machine learning model to a separate computer and/or using the trained machine learning model to generate one or more synthetic medical images of one or more new examination objects.

In another aspect, the present disclosure relates to a computer-implemented method of generating a synthetic image, the method comprising:
- providing a trained machine learning model,
   ∘ wherein the trained machine learning model is configured and was trained on training data to generate a synthetic image based on input data,
   ∘ wherein the training data included, for each examination object of a plurality of examination objects, (i) input data and (ii) target data, wherein the target data included a target image of an examination region of the examination object,
   ∘ wherein training of the trained machine learning model included, for each examination object, the steps:
      ▪ determining target image features based in the target image,
      ▪ inputting the input data into the machine learning model,
      ▪ receiving a synthetic image as an output of the machine learning model,
      ▪ determining image features based on the synthetic image,
      ▪ reducing deviations (i) between the synthetic image and the target image and (ii) between the image features of the synthetic image and the target image features by modifying model parameters,
- receiving new input data of a new examination object,
- inputting the new input data into the trained machine learning model,
- receiving a new synthetic image as an output of the trained machine learning model,
- outputting and/or storing the new synthetic image and/or to transferring the new synthetic image to a separate computer system.

In another aspect, the present disclosure provides a computer system comprising:
a processor; and
a storage medium that stores an application program configured to perform an operation when executed by the processor, said operation comprising the steps of:
   - providing a machine learning model, wherein the machine learning model is configured to generate a synthetic image based on input data and parameters of the machine learning model,
   - providing training data, wherein the training data comprises, for each examination object of a plurality of examination objects, (i) input data and (ii) target data, wherein the target data comprises a target image of an examination region of the examination object,
   - for each target image: determining target image features based on the target image,
   - training the machine learning model, wherein the training comprises, for each examination object of the plurality of examination objects:
      ∘ inputting the input data into the machine learning model,
      ∘ receiving a synthetic image as an output of the machine learning model,
      ∘ determining image features based on the synthetic image,
      ∘ reducing deviations (i) between the synthetic image and the target image and (ii) between the image features of the synthetic image and the target image features by modifying model parameters,
   - outputting and/or storing the trained machine learning model and/or transferring the trained machine learning model to a separate computer and/or using the trained machine learning model to generate one or more synthetic medical images of one or more new examination objects.

In another aspect, the present disclosure provides a computer system comprising:
a processor; and
a storage medium that stores an application program configured to perform an operation when executed by the processor, said operation comprising the steps of:
   - providing a trained machine learning model,
      ∘ wherein the trained machine learning model is configured and was trained on training data to generate a synthetic image based on input data,
      ∘ wherein the training data included, for each examination object of a plurality of examination objects, (i) input data and (ii) target data, wherein the target data included a target image of an examination region of the examination object,
      ∘ wherein training of the trained machine learning model included, for each examination object, the steps:
         ▪ determining target image features based in the target image,
         ▪ inputting the input data into the machine learning model,
         ▪ receiving a synthetic image as an output of the machine learning model,
         ▪ determining image features based on the synthetic image,
         ▪ reducing deviations (i) between the synthetic image and the target image and (ii) between the image features of the synthetic image and the target image features by modifying model parameters,
   - receiving new input data of a new examination object,
   - inputting the new input data into the trained machine learning model,
   - receiving a new synthetic image as an output of the trained machine learning model,
   - outputting and/or storing the new synthetic image and/or to transferring the new synthetic image to a separate computer system.

In another aspect, the present disclosure provides a non-transitory computer readable storage medium having stored thereon software instructions that, when executed by a processor of a computer system, cause the computer system to execute the following steps:
- providing a machine learning model, wherein the machine learning model is configured to generate a synthetic image based on input data and parameters of the machine learning model,
- providing training data, wherein the training data comprises, for each examination object of a plurality of examination objects, (i) input data and (ii) target data, wherein the target data comprises a target image of an examination region of the examination object,
- for each target image: determining target image features based on the target image,
- training the machine learning model, wherein the training comprises, for each examination object of the plurality of examination objects:
   ∘ inputting the input data into the machine learning model,
   ∘ receiving a synthetic image as an output of the machine learning model,
   ∘ determining image features based on the synthetic image,
   ∘ reducing deviations (i) between the synthetic image and the target image and (ii) between the image features of the synthetic image and the target image features by modifying model parameters,
- outputting and/or storing the trained machine learning model and/or transferring the trained machine learning model to a separate computer and/or using the trained machine learning model to generate one or more synthetic medical images of one or more new examination objects.

In another aspect, the present disclosure provides a non-transitory computer readable storage medium having stored thereon software instructions that, when executed by a processor of a computer system, cause the computer system to execute the following steps:
- providing a trained machine learning model,
   ∘ wherein the trained machine learning model is configured and was trained on training data to generate a synthetic image based on input data,
   ∘ wherein the training data included, for each examination object of a plurality of examination objects, (i) input data and (ii) target data, wherein the target data included a target image of an examination region of the examination object,
   ∘ wherein training of the trained machine learning model included, for each examination object, the steps:
      ▪ determining target image features based in the target image,
      ▪ inputting the input data into the machine learning model,
      ▪ receiving a synthetic image as an output of the machine learning model,
      ▪ determining image features based on the synthetic image,
      ▪ reducing deviations (i) between the synthetic image and the target image and (ii) between the image features of the synthetic image and the target image features by modifying model parameters,
- receiving new input data of a new examination object,
- inputting the new input data into the trained machine learning model,
- receiving a new synthetic image as an output of the trained machine learning model,
- outputting and/or storing the new synthetic image and/or to transferring the new synthetic image to a separate computer system.

### BRIEF DESCRIPTION OF THE DRAWINGS

Fig. 1 shows an exemplary and schematic embodiment of the computer-implemented method for training a machine learning model.
Fig. 2 shows an exemplary and schematic embodiment of the computer-implemented method for generating a synthetic image using a trained machine learning model.
Fig. 3 shows another exemplary and schematic embodiment of the computer-implemented method for training a machine learning model.
Fig. 4 shows another exemplary and schematic embodiment of the computer-implemented method for generating a synthetic image using a trained machine learning model.
Fig. 5 shows by way of example and in schematic form a computer system according to the present disclosure.
Fig. 6 shows by way of example and in schematic form a further embodiment of the computer system.
Fig. 7 shows schematically in the form of a flow chart a preferred embodiment of the computer-implemented method for generating a synthetic representation.

### DETAILED DESCRIPTION

The subject matters of the present disclosure will be more particularly elucidated below, without distinguishing between the subject matters (methods, computer systems, storage media). Rather, the elucidations that follow are intended to apply by analogy to all subject matters, irrespective of the context (methods, computer systems, storage media) in which they occur.

Where steps are stated in an order in the present description or in the claims, this does not necessarily mean that this disclosure is limited to the order stated. Instead, it is conceivable that the steps are also executed in a different order or else in parallel with one another, the exception being when one step builds on another step, thereby making it imperative that the step building on the previous step be executed next (which will however become clear in the individual case). The stated orders thus constitute preferred embodiments.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

Unless defined otherwise, all technical and scientific terms used herein have the same meaning as commonly understood by one of ordinary skill in the art.

As used herein, the articles "a" and "an" are intended to include one or more items and may be used interchangeably with "one or more" and "at least one." As used in the description and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise.

The present disclosure provides means for generating a synthetic image.

The term "synthetic" means that the image is not the result of a physical measurement on a real object under examination, but that the image has been generated by a machine learning model. A synonym for the term "synthetic" is the term "artificial". A synthetic image may however be based on one or more measured images, i.e., the machine learning model may be able to generate the synthetic image based on one or more measured images (and/or other/further data).

The synthetic image represents an examination region of an examination object.

The "examination object" is preferably a living being, more preferably a mammal, most preferably a human.

The "examination region" is a part of the examination object, for example an organ or part of an organ or a plurality of organs or another part of the examination object.

For example, the examination region may be a liver, kidney, heart, lung, brain, stomach, bladder, prostate, intestine, thyroid, eye, breast or a part of said parts or another part of the body of a mammal (for example a human).

In one embodiment, the examination region includes a liver or part of a liver or the examination region is a liver or part of a liver of a mammal, preferably a human.

In a further embodiment, the examination region includes a brain or part of a brain or the examination region is a brain or part of a brain of a mammal, preferably a human.

In a further embodiment, the examination region includes a heart or part of a heart or the examination region is a heart or part of a heart of a mammal, preferably a human.

In a further embodiment, the examination region includes a thorax or part of a thorax or the examination region is a thorax or part of a thorax of a mammal, preferably a human.

In a further embodiment, the examination region includes a stomach or part of a stomach or the examination region is a stomach or part of a stomach of a mammal, preferably a human.

In a further embodiment, the examination region includes a pancreas or part of a pancreas or the examination region is a pancreas or part of a pancreas of a mammal, preferably a human.

In a further embodiment, the examination region includes a kidney or part of a kidney or the examination region is a kidney or part of a kidney of a mammal, preferably a human.

In a further embodiment, the examination region includes one or both lungs or part of a lung of a mammal, preferably a human.

In a further embodiment, the examination region includes a thyroid or part of a thyroid of a mammal, preferably a human.

In a further embodiment, the examination region includes an eye or part of an eye of a mammal, preferably a human.

In a further embodiment, the examination region includes a breast or part of a breast or the examination region is a breast or part of a breast of a female mammal, preferably a female human.

In a further embodiment, the examination region includes a prostate or part of a prostate or the examination region is a prostate or part of a prostate of a male mammal, preferably a male human.

The term "image" as used herein means preferably a data structure that represents a spatial distribution of a physical signal. The spatial distribution may be of any dimension, for example 2D, 3D, 4D or any higher dimension. The spatial distribution may be of any shape, for example forming a grid and thereby defining pixels or voxels, the grid being possibly irregular or regular. The physical signal may be any signal, for example proton density, tissue echogenicity, tissue radiolucency, measurements related to the blood flow, information of rotating hydrogen nuclei in a magnetic field, color, level of gray, depth, surface or volume occupancy, such that the image may be a 2D or 3D RGB/grayscale/depth image, or a 3D surface/volume occupancy model. An image is usually composed of discrete image elements (e.g., pixels for 2D images, voxels for 3D images, doxels for 4D images).

The present invention is described in this disclosure predominantly using the example of images representing an examination region of an examination object in real space. However, it should be noted that the present invention can also be applied to representations of an examination region in other spaces, for example, representations of the examination region in frequency space or projection space. In this respect, the term "image" is to be interpreted broadly. In some places in the present disclosure, the term "representation" is used instead of the term "image". However, the terms "representation" and "image" are used interchangeably in the present disclosure.

The "real space" is the three-dimensional Euclidean space, which corresponds to the space that we humans experience with our senses and in which we move. A representation in real space is therefore the more familiar representation for humans.

In a representation in real space, the examination region is usually represented by a plurality of image elements (for example pixels or voxels or doxels), which may for example be in a raster arrangement in which each image element represents a part of the examination region, wherein each image element may be assigned a colour value or grey value. The colour value or grey value represents a signal intensity, for example the attenuation of X-rays.

In a representation in frequency space, the examination region is represented by a superposition of fundamental vibrations. For example, the examination region may be represented by a sum of sine and cosine functions having different amplitudes, frequencies, and phases. The amplitudes and phases may be plotted as a function of the frequencies, for example, in a two- or three-dimensional plot. Usually, the lowest frequency (origin) is placed in the centre. The further away from this centre, the higher the frequencies. Each frequency can be assigned an amplitude representing the frequency in the frequency-space representation and a phase indicating the extent to which the respective vibration is shifted towards a sine or cosine vibration. The k-space data produced by MRI is an example of a representation in frequency space.

A representation in real space can for example be converted (transformed) by a Fourier transform into a representation in frequency space. Conversely, a representation in frequency space can for example be converted (transformed) by an inverse Fourier transform into a representation in real space.

Details about real-space depictions and frequency-space depictions and their respective interconversion are described in numerous publications, see for example https://see .stanford.edu/materials/lsoftaee261/book-fall-07.pdf.

A representation of an examination region in the projection space is usually the result of a computed tomography examination prior to image reconstruction. In other words: the raw data obtained in the computed tomography examination can be understood as a projection-space representation. In computed tomography, the intensity or attenuation of X-radiation as it passes through the examination object is measured. From this, projection values can be calculated. In a second step, the object information encoded by the projection is transformed into an image (e.g., a real-space representation) through a computer-aided reconstruction. The reconstruction can be effected with the Radon transform. The Radon transform describes the link between the unknown examination object and its associated projections.

Details about the transformation of projection data into a real-space representation are described in numerous publications, see for example K. Catch: The Radon Transformation and Its Application in Tomography, Journal of Physics Conference Series 1903(1):012066.

Preferably the synthetic image is a synthetic medical image.

A "medical image" is a preferably visual representation of the human body or a part thereof or a visual representation of the body of an animal or a part thereof. Medical images can be used, e.g., for diagnostic and/or treatment purposes.

Techniques for generating medical images include X-ray radiography, computerized tomography, fluoroscopy, magnetic resonance imaging, ultrasonography, endoscopy, elastography, tactile imaging, thermography, microscopy, positron emission tomography, optical coherence tomography, fundus photography, and others.

Examples of medical images include CT (computer tomography) scans, X-ray images, MRI (magnetic resonance imaging) scans, PET (positron emission tomography) scans, fluorescein angiography images, OCT (optical coherence tomography) scans, histological images, ultrasound images, fundus images and/or others.

In an embodiment of the present disclosure, the medical image is a radiologic image. "Radiology" is the branch of medicine concerned with the application of electromagnetic radiation and mechanical waves (including, for example, ultrasound diagnostics) for diagnostic, therapeutic and/or scientific purposes. In addition to X-rays, other ionizing radiation such as gamma rays or electrons are also used. Since a primary purpose is imaging, other imaging procedures such as sonography and magnetic resonance imaging (MRI) are also included in radiology, although no ionizing radiation is used in these procedures. Thus, the term "radiology" as used in the present disclosure includes, in particular, the following examination procedures: computed tomography, magnetic resonance imaging, sonography, positron emission tomography.

The radiologic image can be, e.g., a 2D or 3D CT scan or MRI scan. The radiologic image may be a representation of an examination region generated with or without a contrast agent.

"Contrast agents" are substances or mixtures of substances that improve the depiction of structures and functions of the body in radiological examinations.

In computed tomography, iodine-containing solutions are usually used as contrast agents. In magnetic resonance imaging (MRI), superparamagnetic substances (for example iron oxide nanoparticles, superparamagnetic iron-platinum particles (SIPPs)) or paramagnetic substances (for example gadolinium chelates, manganese chelates, hafnium chelates) are usually used as contrast agents. In the case of sonography, liquids containing gas-filled microbubbles are usually administered intravenously. Examples of contrast agents can be found in the literature (see for example A.S.L. Jascinth et al.: Contrast Agents in computed tomography: A Review, Journal of Applied Dental and Medical Sciences, 2016, vol. 2, issue 2, 143-149; H. Lusic et al.: X-ray-Computed Tomography Contrast Agents, Chem. Rev. 2013, 113, 3, 1641-1666; https://www.radiology.wisc.edu/wp-content/uploads/2017/10/contrast-agents-tutorial.pdf, M.R. Nouh et al.: Radiographic and magnetic resonances contrast agents: Essentials and tips for safe practices, World J Radiol. 2017 Sep. 28; 9(9): 339-349; L.C. Abonyi et al.: Intravascular Contrast Media in Radiography: Historical Development & Review of Risk Factors for Adverse Reactions, South American Journal of Clinical Research, 2016, vol. 3, issue 1, 1-10; ACR Manual on Contrast Media, 2020, ISBN: 978-1-55903-012-0; A. Ignee et al.: Ultrasound contrast agents, Endosc Ultrasound. 2016 Nov-Dec; 5(6): 355-362).

The synthetic image is generated with the help of a trained machine learning model.

Such a "machine learning model", as used herein, may be understood as a computer implemented data processing architecture. The machine learning model can receive input data and provide output data based on that input data and on parameters of the machine learning model (model parameters). The machine learning model can learn a relation between input data and output data through training. In training, parameters of the machine learning model may be adjusted in order to provide a desired output for a given input.

The process of training a machine learning model involves providing a machine learning algorithm (that is the learning algorithm) with training data to learn from. The term "trained machine learning model" refers to the model artifact that is created by the training process. The training data must contain the correct answer, which is referred to as the target. The learning algorithm finds patterns in the training data that map input data to the target, and it outputs a trained machine learning model that captures these patterns.

In the training process, input data are inputted into the machine learning model and the machine learning model generates an output. The output is compared with the (known) target. Parameters of the machine learning model are modified in order to reduce the deviations between the output and the (known) target to a (defined) minimum.

In general, a loss function can be used for training, where the loss function can quantify the deviations between the output and the target.

The aim of the training process can be to modify (adjust) parameters of the machine learning model in order to reduce the loss to a (defined) minimum.

In the case of the present disclosure, a machine learning model is trained to generate synthetic images.

In a first step, the machine learning model is provided.

The machine learning model is configured to generate a synthetic image based on input data and parameters of the machine learning model.

The machine learning model can be or include an artificial neural network.

An "artificial neural network" (ANN) is a biologically inspired computational model. An ANN usually comprises at least three layers of processing elements: a first layer with input neurons (nodes), a k^{th} layer with output neurons, and k-2 inner layers, where k is a natural number greater than 2.

In such a network, the input neurons serve to receive the input data. If the input data constitute or comprise an n-dimensional vector (e.g., a feature vector), with n being an integer equal to or greater than 1, there is usually one input neuron for each component of the vector. If the input data constitute or comprise an image, there is usually one input neuron for each colour/greyscale channel of each image element. The output neurons serve to output a synthetic image. The processing elements of the layers are interconnected in a predetermined pattern with predetermined connection weights therebetween. Each network node represents a pre-defined calculation of the weighted sum of inputs from prior nodes and a non-linear output function. The combined calculation of the network nodes relates the inputs to the outputs. It should be noted that an ANN can also comprise connection biases.

The machine learning model can be or comprise a convolutional neural network (CNN). A "CNN" is a class of deep neural networks, most commonly applied to processing images. A CNN comprises an input layer with input neurons, an output layer with output neurons, as well as multiple hidden layers between the input layer and the output layer.

The nodes in the CNN input layer are usually organized into a set of "filters" (feature detectors), and the output of each set of filters is propagated to nodes in successive layers of the network. The computations for a CNN include applying the convolution mathematical operation to each filter to produce the output of that filter. Convolution is a specialized kind of mathematical operation performed by two functions to produce a third function that is a modified version of one of the two original functions. In convolutional network terminology, the first function to the convolution can be referred to as the input, while the second function can be referred to as the convolution kernel. The output may be referred to as the feature map. For example, the input to a convolution layer can be a multidimensional array of data that defines the various colour/greyscale components of an input image. The convolution kernel can be a multidimensional array of parameters, where the parameters are adapted by the training process for the neural network.

The objective of the convolution operation is to extract features (such as, e.g., edges from an input image). Conventionally, the first convolutional layer is responsible for capturing the low-level features such as edges, colour, gradient orientation, etc. With added layers, the architecture adapts to the high-level features as well, giving a network which has the wholesome understanding of images in the dataset. Similar to the convolutional layer, the pooling layer is responsible for reducing the spatial size of the feature maps. It is useful for extracting dominant features with some degree of rotational and positional invariance, thus maintaining the process of effectively training of the model. Adding a fully-connected layer is a way of learning non-linear combinations of the high-level features as represented by the output of the convolutional part.

The machine learning model can have an autoencoder architecture.

An "autoencoder" is a type of neural network that is often used for unsupervised learning and dimensionality reduction. An autoencoder is designed to learn a compressed representation (encoding) of the input data and subsequently reconstruct (decode) the original input as accurately as possible or generate an output derived from the input data.

An autoencoder usually consists of two main components: the encoder and the decoder. The encoder maps the input data to a lower-dimensional space, typically referred to as the latent space or bottleneck. The dimensionality of this latent space is usually smaller than the dimensionality of the input data, effectively compressing the information. The decoder then reconstructs the original input from the encoded representation using another neural network, mirroring the encoder's structure. However, the decoder can also be used to generate a different output from the encoded representation, e.g., a synthetic image.

The machine learning model can have a U-Net architecture.

U-Net refers to a convolutional neural network (CNN) architecture that was introduced by Ronneberger *et al.* in 2015 (see, e.g., O. Ronneberger et al.: U-Net: Convolutional Networks for Biomedical Image Segmentation, arXiv:1505.04597v1).

The U-Net architecture is notable for its symmetric and U-shaped structure, which inspired its name. It is designed to capture both local and global information while maintaining fine-grained spatial resolution. The U-Net architecture typically consists of two main parts: the contracting path (encoder) and the expansive path (decoder). The contracting path in the U-Net architecture resembles a traditional CNN, where each layer consists of convolutional and pooling operations. As the name suggests, this part of the network is responsible for capturing the low-level and high-level features, gradually reducing the spatial resolution and increasing the number of feature channels.

The expansive path in the U-Net architecture, which is the decoder part, performs a series of upscaling operations to gradually restore the spatial resolution of the encoded features. The upsampling is typically achieved using transpose convolutions or other upsampling techniques. Additionally, at each layer in the expansive path, skip connections are used to concatenate feature maps from the corresponding layer in the contracting path. These skip connections help in preserving low-level and fine-grained spatial details, aiding in accurate segmentation.

The machine learning model can be or comprise a transformer model.

At the core of such a transformer model is the transformer architecture, which relies heavily on self-attention mechanisms to process sequential data efficiently.

Transformer models use self-attention mechanisms to capture contextual relationships between portions of the input data in a sequence. This enables transformer models to model long-range dependencies effectively, allowing them to produce more accurate predictions.

The transformer architecture consists of two main components: the encoder and the decoder. The encoder processes the input sequence, modeling its contextual relationships, while the decoder generates the output sequence based on the encoded information. Both the encoder and decoder are composed of multiple layers of self-attention mechanisms and feed-forward neural networks. Details about transformers may be found in scientific publication (see, e.g., S. Khan et al.: Transformers in Vision: A Survey, arXiv:2101.01169v5).

As already mentioned, the machine learning model of the present disclosure is configured to generate a synthetic image based on input data and parameters of the machine learning model.

The input data may comprise one or more images, in particular medical images.

In other words, the machine learning model can be configured and trained to generate a synthetic image based on one or more images (input images). Each image may represent the examination region of the examination object.

The one or more images of the input data can be measured images, i.e., images that are the result of a measurement on an examination object.

The one or more images of the input data can be one or more medical images.

However, it is also possible for the synthetic image to be generated on other or additional input data.

Such additional input data can comprise additional images, such as one or more images adjacent to one or more input images.

Such other/additional input data can be information about the examination object, the examination region and/or the (measurement) method used to generate the input data and/or the target data.

If the examination object is a patient, the other/additional data can be patient data, for example.

Patient data may include, e.g.: demographic data (age, sex, body size (height), body weight, body mass index, ethnicity), resting heart rate, heart rate variability and other data derived from heart rate, glucose concentration in blood and urine, body temperature, impedance (e.g., thoracic impedance), blood pressure (e.g., systolic and/or diastolic arterial peripheral blood pressure), (estimated) glomerular filtration rate, urine albumin-to-creatinine ratio (uACR), blood measurement values (e.g., blood sugar, oxygen saturation, erythrocyte count, hemoglobin content, leukocyte count, platelet count, inflammation values, blood lipids including low-density lipoprotein cholesterol (LDL) and high-density lipoprotein cholesterol (HDL), ions including Na+ and corrected calcium), pre-existing disease(s), genetic background, lifestyle information about the life of the patient, such as consumption of alcohol, smoking, and/or exercise and/or the patient's diet, information about how long the patient has had one or more diseases (e.g., diabetes, hypertension, and/or cardiovascular disease), medical intervention parameters such as regular medication, occasional medication, or other previous or current medical interventions and/or other information about the patient's previous and/or current treatments and/or reported health conditions and/or combinations thereof.

Patient data may include information from an electronic medical record (EMR, also referred to as electronic health record (EHR)). The EMR may contain information about a hospital's or physician's practice where certain treatments were performed and/or certain tests were performed, as well as various other (meta-)information about the patient's treatments, medications, tests, and physical and/or mental health records.

Patient data may include information about a person's condition obtained from the person himself/herself (self-assessment data, (electronic) patient reported outcome data (e)PRO)).

Patient data can be provided by the patient and/or any other person such as a physician and/or a physician assistant. Patient data may be entered into one or more computer systems by said person or persons via input means (such as a keyboard, a touch-sensitive surface, a mouse, a microphone, and/or the like).

Patient data can be captured (e.g., automatically) by one or more sensors, e.g., blood pressure sensor, motion sensor, activity tracker, blood glucose meter, heart rate meter, thermometer, impedance sensor, microphone (e.g., for voice analysis) and/or others.

Patient data can be measured by a laboratory and stored in a data storage by laboratory personnel.

Patient data can be read from one or more data storages.

In one embodiment of the present disclosure, the machine learning model is configured and trained to generate a synthetic radiologic image representing an examination region of an examination object after application of a second amount of a contrast agent based on one or more radiologic images (and optionally additional input data) representing the examination region of the examination object before and/or after application of a first amount of the contrast agent.

The first amount is different from the second amount. The first amount may be less than the second amount, or the first amount may be greater than the second amount. If the first amount is less than the second amount, the method of the present disclosure may be used to reduce the amount of contrast agent in a radiologic examination (see, e.g., WO2019/074938A1, WO2022184297A1). For example, a first radiologic image of the examination region of an examination object may be generated after application of an amount less than the standard amount, and based on this measured radiologic image, a synthetic image of the examination region of the examination object after application of the standard amount may be generated using the trained machine learning model. For example, a first radiologic image of the examination region of an examination object may be generated without contrast agent, a second radiologic image of the examination region of the examination object may be generated after application of an amount less than the standard amount, and based on this measured radiologic images, a synthetic image of the examination region of the examination object after application of the standard amount may be generated using the trained machine learning model.

The standard amount is the amount recommended by the manufacturer and/or distributor of the contrast agent and/or the amount approved by a regulatory authority and/or the amount listed in a package insert for the contrast agent.

In another embodiment of the present disclosure, the machine learning model is configured and trained to generate a synthetic radiologic image representing an examination region of an examination object after application of a second contrast agent based on one or more radiologic images (and optionally additional input data) representing the examination region of the examination object before and/or after application of a first contrast agent, wherein the first contrast agent and the second contrast agent are different contrast agents (see, e.g., WO2021197996A1).

In another embodiment of the present disclosure, the machine learning model is configured and trained to generate a synthetic high-dose CT image of an examination region of an examination object based on one or more low-dose CT image(s) of the examination region of the examination object. The terms "high-dose" and "low-dose" refer to the radiation dose and have the definitions commonly used in the state of the art (see, e.g., WO2016/175755A1).

In another embodiment of the present disclosure, the machine learning model is configured and trained to generate a synthetic medical image of an examination region of an examination object at a first time point based on one or more medical image(s) of the examination region of the examination object at one or more other time points.

For example, the machine learning model may be configured and trained to generate a synthetic radiologic image representing an examination region of an examination object at a point in time in a dynamic radiologic examination (e.g., a functional magnetic resonance imaging examination). The synthetic radiologic image may be generated based on one or more measured radiologic images (and optionally additional input data) representing the examination region of the examination object at one or more other points in time in the dynamic radiologic examination.

Functional magnetic resonance imaging (fMRI) is a technique used in radiology and neuroscience to measure and map brain activity by detecting changes in blood flow and oxygenation levels. During an fMRI scan, a strong magnetic field and radio waves are used to create images of the brain. By monitoring the changes in blood oxygenation levels, fMRI can indirectly measure neural activity in different regions of the brain. This is based on the principle that increased neural activity in a specific brain area leads to an increased demand for oxygenated blood supply to that region (see, e.g.: Z. Huang et al.: Timescales of Intrinsic BOLD Signal Dynamics and Functional Connectivity in Pharmacologic and Neuropathologic States of Unconsciousness, The Journal of Neuroscience, 2018, 38(9):2304-2317).

For example, the machine learning model can be configured and trained to generate a synthetic radiologic image of an examination region of an examination object at a first time point before or after application of a contrast agent based on one or more radiologic image(s) (and optionally further input data) of the examination region of the examination object at one or more other time points before or after the application of the contrast agent. In this context, the invention can be used to shorten the time an examination object has to spend in an MRI scanner, for example. Instead of measuring MRI images over a longer period of time, MRI images are measured only within a part of the time period and one or more MRI images are predicted for the remaining part of the time period using the trained machine learning model (see, e.g. WO2021052896A1 and WO2021069338A1).

In another embodiment of the present disclosure, the machine learning model is configured and trained to generate a synthetic medical image of a first modality, based on one or more medical image(s) (and optionally additional input data) of one or more other modalities. For example, the machine learning model can be configured and trained to generate a synthetic CT image of an examination region of an examination object based on one or more MRI images of the examination region of the examination object. For example, the machine learning model can be configured and trained to generate a synthetic MRI image of an examination region of an examination object based on one or more CT images of the examination region of the examination object.

In another embodiment of the present disclosure, the machine learning model is configured and trained to generate a synthetic medical image according to a first measurement protocol, based on one or more medical image(s) (and optionally additional input data) according to one or more other measurement protocols. The measurement protocols may be different MRI imaging sequences, for example. For example, the machine learning model can be configured and trained to generate a synthetic T1-weighted MRI image of an examination region of an examination object based on a T2-weighted MRI image of the examination region of the examination object or *vice versa.* Transformations between other measurement protocols such as diffusion weighted imaging (DWI), fluid attenuated inversion recovery (Flair), proton density (PD), and/or others are also possible.

In another embodiment of the present disclosure, the machine learning model is configured and trained to generate a synthetic medical image resulting from applying first measurement parameters based on one or more measured medical images (and optionally additional input data) resulting from applying second measurement parameters. The second measurement parameters differ from the first measurement parameters. Such a trained machine learning model can be used, for example, to harmonize medical images generated at different locations (sites) and/or under different conditions in order to (better) compare them. In other words, harmonization of scan protocols can be achieved by image-to-image translations between medical images created with different parameters (e.g., repetition time (TR), echo time (TE) in case of MRI images) in multi-site datasets.

In a further step, training data is provided. The training data comprises, for each examination object of a plurality of examination objects, (i) input data and (ii) target data, wherein the target data comprises an image of an examination region of the examination object.

The term "plurality" as it is used herein means an integer greater than 1, usually greater than 10, preferably greater than 100.

The input data has already been described above. The target data comprises an image of an examination region of the examination object (target image). The image of the target data (target image) specifies what the synthetic image should look like. In other words, the machine learning model is trained to generate a synthetic image that comes as close as possible to the target image, i.e., has as few deviations as possible from the target image.

The target image is usually (but not necessarily) a measured image.

There may be further target data. For example, the machine learning model can be configured and trained to not only generate a synthetic image based on input data, but also to perform another task that requires additional (further) target data.

In a further step, image features are determined from the target image. These image features of the target image are also referred to as "target image features" in this disclosure.

"Image features" refer to characteristics and/or patterns extracted from images that are used to represent and analyze visual data. These features help in capturing relevant information about the content, style and/or structure of an image.

Examples of commonly used image features are:
Color/greyscale histograms: These features represent the distribution of color/greyscale values in an image, capturing the frequency of different color/greyscale values. They provide information about the overall color/greyscale composition of an image (see, e.g., M. Blachnik et al.: Image classification by histogram features created with Learning Vector Quantization, Proceedings of International Conference on Artificial Neural Networks, ICANN 2008, Volume 5163 of the series Lecture Notes in Computer Science, pp. 827-836).
Texture features: Texture features capture the spatial arrangement and patterns of texture in an image. They can describe properties like roughness, smoothness, or coarseness (see, e.g., L- Armi et al.: Texture Image Analysis and Texture Classification Methods - A Review, International Online Journal of Image Processing and Pattern Recognition, 2019, Vol. 2, No.1, pp. 1-29).
Edge feature: Edge features identify and represent the boundaries or transitions between different regions in an image. They capture changes in pixel/voxel intensity (see, e.g., M. R. Wankhade et al., International Journal of Computer Science and Mobile Computing, 2017, Vol. 6, Issue. 6, pp. 336-345).
SIFT (Scale-Invariant Feature Transform) feature: SIFT features are robust and invariant to changes in scale, rotation, and illumination. They identify key points in an image and describe their local appearance (see, e.g.: D. G. Lowe: Distinctive Image Features from Scale Invariant Keypoints, International Journal of Computer Vision 2004, 60, pp. 91-110).
HOG (Histogram of Oriented Gradients) feature: HOG features capture the distribution of gradient orientations in an image. They are particularly effective for object detection and recognition tasks, as they can represent the shape and structure of objects (see, e.g., W. Zhou et al.: Histogram of Oriented Gradients Feature Extraction From Raw Bayer Pattern Images, IEEE Transactions on Circuits and Systems II: Express Briefs, 2020, pp.1-5).
Convolutional Neural Network (CNN) features: With the advent of deep learning, features extracted from pre-trained CNN models have become popular. These features are learned hierarchically and can capture complex visual patterns and semantics (see, e.g., Y. H. Liu et al.: Feature Extraction and Image Recognition with Convolutional Neural Networks, Journal of Physics Conference Series, 2018, 1087(6): 062032).

Image features can be roughly divided into low-level image features and high-level image features (see, e.g., T. G. Nisia and S. Rajesh: Extraction of High-level and Low-level feature for classification of Image using Ridgelet and CNN based Image Classification, Journal of Physics: Conference Series, 2021, 1911. 012019). Both can be used to characterize an image.

Low-level image features, also known as primitive features, are basic and local characteristics that can be extracted directly from the pixel/voxel values of an image. They typically capture low-level visual properties such as color/greyscales, texture, edges, and gradients. Examples of low-level image features include color histograms, edge detectors, and texture descriptors. These features provide fundamental information about the image's visual content but may lack semantic meaning or higher-level context.

On the other hand, high-level image features are more abstract and capture complex patterns and semantics within an image. They are derived from lower-level features and often involve more sophisticated processing techniques. High-level features can represent objects, scenes, or concepts in an image. They encode higher-level information such as shapes, structures, or relationships between objects. Examples of high-level image features include object detectors, semantic segmentation maps, or features extracted from deep convolutional neural networks (CNNs). These features are learned through training on large datasets and can capture rich semantic information, enabling tasks like object recognition, scene understanding, and image captioning.

Image features can also be roughly divided into content features and style features (see, e.g. J. B. Tenenbaum, W. T. Freeman: Separating style and content with bilinear models, Neural Computation, 2020, 12, pp. 1247-1283; A. Elgammal, C.-S. Lee: Separating style and content on a nonlinear manifold, Proceedings of the 2004 IEEE Computer Society Conference on Computer Vision and Pattern Recognition, 2004, pp. I-I). Both can be used to characterize an image.

Content features capture the underlying objects, shapes, and structures within an image. They represent the high-level information that defines what is present in the image (e.g., objects and their arrangements). These features are typically derived by extracting feature map activations from deeper layers of deep convolutional neural networks (CNNs) trained on large datasets. By extracting content features, a model can understand and recognize objects, scenes, and other semantic elements in an image.

Style features, on the other hand, capture the artistic or visual style of an image. They represent the texture, colors, patterns, and other aesthetic properties that define the unique style or artistic characteristics of an image. These features are typically extracted from feature map activations of earlier layers of the CNN, and computed via a Gram matrix. The Gram matrix doesn't hold onto spatial information (where objects are located in the image) but captures how often different features appear together and the prevalence of certain features. This defines the "style" of the image and captures patterns, textures, and the general essence of the image's style but not the specific arrangement of objects.

By separating content and style features, models can disentangle the underlying content of an image from its artistic expression. This separation enables various applications, such as transferring the style of one image onto the content of another, generating new images with a specific style, or analyzing and classifying images based on their artistic characteristics.

Preferably, the image features are or comprise one or more feature maps.

As described above, such feature maps can be generated with the help of a pre-trained convolutional neural network.

In the case of a convolutional neural network, a feature map refers to the output produced by a layer in the network. It represents the activation or response of a group of neurons, also called a feature detector, to a specific pattern or feature within the input data.

In CNNs, feature maps are usually generated through the application of convolutional filters to the input data. Each filter scans the input data and performs a convolution operation by taking the dot product between the filter weights and a portion of the input data. This operation produces a scalar value, also known as an activation or feature response, which indicates the presence or strength of the detected feature at that particular spatial location in the input data.

A feature map usually consists of multiple activation values, one for each location in the input data, usually forming a spatial grid. Each individual activation value represents the response of the corresponding feature detector to a specific pattern or feature within the input data. As the network architecture becomes deeper, multiple layers of feature maps are generated by applying different sets of filters, each specializing in detecting specific patterns or features.

Feature maps serve as intermediate representations within the neural network, which subsequently undergo additional operations such as pooling, activation functions, and dense layers to extract and refine more complex representations.

In a further step, the machine learning model is trained using the training data.

In such a training, the input data for each examination object of the plurality of examination objects is inputted into the machine learning model. The machine learning model generates a synthetic image based on the input data and model parameters.

From the synthetic image, image features are determined. Typically, the image features of the synthetic image are generated in the same way as the target image features of the target image, so that there is an image feature of the synthetic image for each target image feature.

In a next step, (i) the synthetic image is compared with the target image and (ii) the image features of the synthetic image are compared with the target image features.

Deviations (i) between the synthetic image and the target image and (ii) between the image features of the synthetic image and the target image features are determined.

The deviations can be quantified using one or more loss functions.

For example, the deviations between the synthetic image and the target image can be quantified by determining the absolute differences in color value/grey values element by element and adding them up across all image elements.

The deviations between the image features of the synthetic image and the target image features can be quantified in an analogous way. If single values are involved, the absolute difference between the single values can be calculated. In the case of vectors, matrices or tensors, the absolute differences can be calculated element by element and added across all elements.

Finally, the deviations (i) between synthetic image and the target image and (ii) between the image features of the synthetic images and the target image features can be combined in a joint loss function, e.g., by addition.

The aim of the training can be to reduce the deviations by minimizing the loss function. The loss function is usually minimized in an optimization process (e.g., a gradient descent process) by modifying the model parameters.

Training can be carried out until a stop criterion is met.

Such a stop criterion can be for example: a predefined maximum number of training steps/epochs has been performed, deviations between output data and target data can no longer be reduced by modifying the model parameters, a predefined minimum of the loss function is reached, and/or an extreme value (e.g., maximum or minimum) of another performance value is reached.

The trained machine learning model and/or the modified parameters of the trained machine learning model may be output, stored and/or transmitted to a separate computer system.

The trained machine learning model can be used for inference. "Inference" is understood as the generation of a synthetic image of the examination region of a new examination object. The term "new" means that data from the new examination object has usually not already been used when training the machine learning model.

New input data is received to generate a synthetic representation of the examination region of the new examination object. The new input data of the new examination object usually corresponds to the input data of the plurality of examination objects that were used to train the machine learning model.

The input data is inputted into the trained machine learning model. A synthetic image is received as output from the trained machine learning model. The synthetic image represents the examination region of the new examination object.

The synthetic image can be outputted, stored and/or transmitted to a separate computer system.

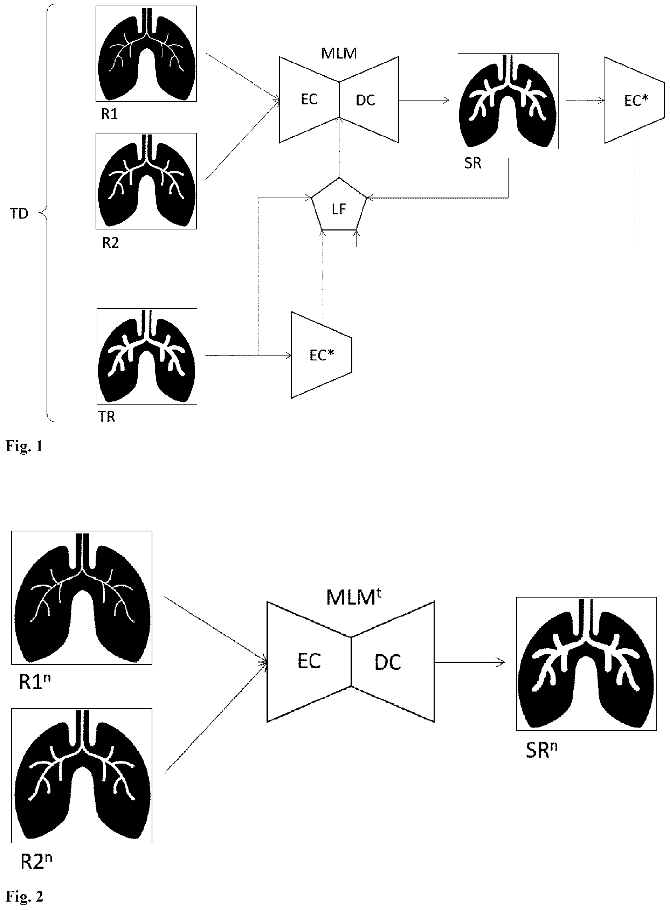

Fig. 1 shows an exemplary and schematic embodiment of the computer-implemented method for training a machine learning model. The training method generates a model that can be used to generate synthetic images of an examination region, which is why the method can also be referred to as a method for generating a model for generating synthetic images of an examination region.

The machine learning model MLM shown in Fig. 1 is trained on training data. The training data comprises, for each examination object of a plurality of examination objects, (i) input data and (ii) target data.

In the example shown in Fig. 1, only one data set TD of one examination object is shown. This data set TD comprises a first representation R1 of a human lung and a second representation R2 of the human lung as input data, as well as a third representation TR of the human lung as target data. The third representation TR is also referred to here as the target representation.

The first representation R1 may be a radiologic image, such as a CT image or an MRI image. The first representation R1 may represent the human lung without a contrast agent.

The second representation R2 may also be a radiologic image, such as a CT image or an MRI image. The second representation R2 may represent the human lung after application of a first amount of a contrast agent.

The target representation TR may also be a radiologic image, such as a CT image or an MRI image. The target representation TR may represent the human lung after application of a second amount of a contrast agent. The second amount differs from the first amount. For example, the second amount may be greater than the first amount.

The first representation R1 and the second representation R2 are inputted into the machine learning model MLM. The machine learning model MLM comprises an encoder EC and a decoder DC. The encoder EC is configured to extract features from the first representation R1 and the second representation R2 and to generate a feature vector based on the first representation R1 and the second representation R2. The decoder DC is configured to generate a synthetic representation SR based on the feature vector. The feature vector is not explicitly shown in Fig. 1.

The synthetic representation SR may be a synthetic radiologic image, such as a synthetic CT image or a synthetic MRI image. The synthetic representation SR may represent the human lung after application of the second amount of a contrast agent.

In other words, the machine learning model MLM may be configured and trained to generate a synthetic representation SR that looks like the target representation TR based on the first representation R1 and the second representation R2 and parameters of the machine learning model MLM.

The synthetic representation SR is fed to an additional encoder EC*. The additional encoder EC* is configured to extract image features from the synthetic representation SR.

The additional encoder EC* can, for example, be an encoder of a pre-trained autoencoder that contains a CNN. The features can, for example, be feature maps that are generated by the CNN.

The additional encoder EC* can also be the encoder EC of the machine learning model MLM or be based on it or replaced by it in the course of training.

Similarly, the target representation TR is also fed to the additional encoder EC*. For better clarity, the additional encoder EC* is shown twice in Fig. 1, but it is the same additional encoder EC*.

The additional encoder EC* therefore also extracts image features from the target representation TR.

A loss function LF is used to quantify (i) deviations between the synthetic representation SR and the target representation TR and (ii) deviations between the features extracted from the synthetic representation SR and the features extracted from the target representation TR.

By modifying parameters of the machine learning model MLM in an optimization procedure (e.g., a gradient descent procedure), the deviations can be reduced. The above steps are repeated for a plurality of input data and target data of a plurality of human lungs until a stop criterion is met.

As already described, additional input data can be used to train the machine learning model MLM.

Such additional input data can comprise additional representations, such as one or more representations of the examination object which represent areas adjacent to the examination region represented by the representations R1 and/or R2.

Such other/additional input data can be information about the examination object, the examination region and/or the (measurement) method used to generate the input data and/or the target data.

Once the machine learning model has been trained, it can be used to predict, i.e., generate a synthetic representation of a new human lung. The term "new" means that no representation of the human lung was usually used when training the machine learning model.

Fig. 2 shows an exemplary and schematic embodiment of the computer-implemented method for generating a synthetic representation using a trained machine learning model.

The trained machine learning model MLM^{t} shown in Fig. 2 may have been trained in a method as described with respect to Fig. 1.

In a first step, a first representation R1ⁿ and a second representation R2ⁿ are received. It is possible that additional input data is received.

The first representation R1ⁿ may be a radiologic image, such as a CT image or an MRI image. The first representation R1ⁿ may represent a human lung without a contrast agent.

The second representation R2ⁿ may also be a radiologic image, such as a CT image or an MRI image. The second representation R2ⁿ may represent the human lung after application of a first amount of a contrast agent.

The first representation R1ⁿ and the second representation R2ⁿ are inputted into the trained machine learning model MLM^{t}.

The trained machine learning model MLM^{t} is configured and was trained to generate a synthetic representation SRⁿ based on the first representation R1ⁿ and the second representation R2ⁿ.

The synthetic representation SRⁿ may be a synthetic radiologic image, such as a synthetic CT image or a synthetic MRI image. The synthetic representation SR represents the human lung after application of a second amount of a contrast agent. The second amount differs from the first amount. For example, the second amount may be greater than the first amount (this depends on what the machine learning model MLM^{t} was trained for).

The synthetic representation SRⁿ can be outputted (e.g., displayed on a screen and/or printed using a printer) and/or stored in a data memory and/or transmitted to a separate computer system.

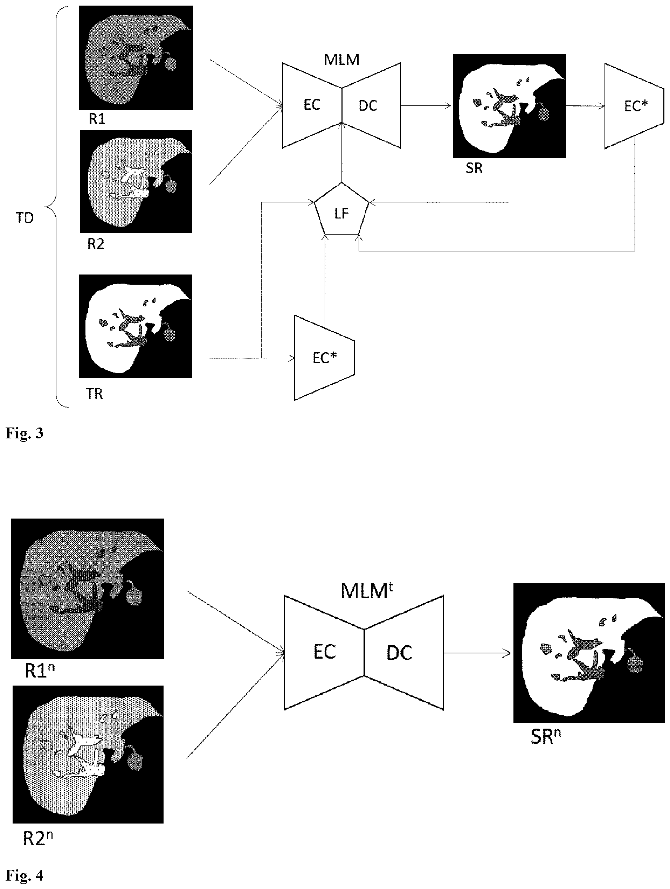

Fig. 3 shows another exemplary and schematic embodiment of the computer-implemented method for training a machine learning model. The training method generates a model that can be used to generate synthetic images of an examination region, which is why the method can also be referred to as a method for generating a model for generating synthetic images of an examination region.

The machine learning model MLM shown in Fig. 3 is trained on training data. The training data comprises, for each examination object of a plurality of examination objects, (i) input data and (ii) target data.

In the example shown in Fig. 3, only one data set TD of one examination object is shown. This data set TD comprises a first representation R1 of a human liver and a second representation R2 of the human liver as input data, as well as a third representation TR of the human liver as target data. The third representation TR is also referred to here as the target representation.

The first representation R1 may be a radiologic image, such as a CT image or an MRI image. The first representation R1 may represent the human liver at a first time point before or after application of a contrast agent.

The second representation R2 may also be a radiologic image, such as a CT image or an MRI image. The second representation R2 may represent the human liver at a second time point before or after application of the contrast agent.

The target representation TR may also be a radiologic image, such as a CT image or an MRI image. The target representation TR may represent the human liver at a third time point before or after application of the contrast agent.

The first point in time, the second point in time and the third point in time are different points in time. For example, the second point in time can follow the first point in time and the third point in time can follow the second point in time. However, it is also possible that the second time point follows the third time point and the first time point follows the second time point.

The contrast agent can be a hepatobiliary contrast agent. A hepatobiliary contrast agent has the characteristic features of being specifically taken up by liver cells (hepatocytes), accumulating in the functional tissue (parenchyma) and enhancing contrast in healthy liver tissue. An example of a hepatobiliary MRI contrast agent is the disodium salt of gadoxetic acid (Gd-EOB-DTPA disodium), which is described in US Patent No. 6 039 931A and is commercially available under the trade names Primovist^{®} and Eovist^{®}. Further hepatobiliary contrast agents are described inter alia in WO2022/194777.

It should be noted that such a hepatobiliary MRI contrast agent is not limited to use in an MRI examination. It is possible that such a hepatobiliary MRI contrast agent is also used in a CT examination. In a CT examination, such a hepatobiliary MRI contrast agent will of course produce less contrast than a CT contrast agent. However, no hepatobiliary CT contrast agents are currently approved for CT examinations. Therefore, the present invention can also be used to increase the contrast of an MRI contrast agent in a CT examination and to utilize the hepatobiliary properties of a hepatobiliary MRI contrast agent in CT examinations.

For example, the first representation R1 can represent a human liver in the native phase, i.e. without contrast agent.

The second representation R2 may represent the human liver at a first time point after administration of a (e.g., hepatobiliary) contrast agent. The second representation R2 may represent the human liver, e.g., in the arterial phase, the portal venous phase or the transitional phase after administration of a hepatobiliary contrast agent. The target representation TR may represent the human liver, e.g., in the hepatobiliary phase after administration of the hepatobiliary contrast agent.

These phases are described, for example, in: C. L. Hui et al.: Patterns of enhancement in the hepatobiliary phase of gadoxetic acid-enhanced MRI, Br J Radiol., 2020, 93: 20190989.

It is also possible that, in addition to the first representation R1 and the second representation R2, there are one or more further representations that represent the human liver at further points in time before and/or after the application of a (e.g., hepatobiliary) contrast agent.

It is also possible for the input data to comprise only one representation, e.g., only the first representation R1 or only the second representation R2.

In the example shown in Fig. 3 the first representation R1 and the second representation R2 are inputted into the machine learning model MLM. The machine learning model MLM comprises an encoder EC and a decoder DC. The encoder EC is configured to extract features from the first representation R1 and the second representation R2 and to generate a feature vector based on the first representation R1 and the second representation R2. The decoder DC is configured to generate a synthetic representation SR based on the feature vector. The feature vector is not explicitly shown in Fig. 3.

The machine learning model MLM is configured and trained to generate a synthetic representation SR that looks like the target representation TR based on the first representation R1 and the second representation R2 and parameters of the machine learning model MLM.

Thus, the synthetic representation SR may represent the human lung at a third time point before or after application of the contrast agent, e.g., in the hepatobiliary phase after administration of a hepatobiliary contrast agent.

The synthetic representation SR is fed to an additional encoder EC*. The additional encoder EC* is configured to extract image features from the synthetic representation SR.

The additional encoder EC* can, for example, be an encoder of a pre-trained autoencoder that contains a CNN. The features can, for example, be feature maps that are generated by the CNN.

The additional encoder EC* can also be the encoder EC of the machine learning model MLM or be based on it or replaced by it in the course of training.

Similarly, the target representation TR is also fed to the additional encoder EC*. For better clarity, the additional encoder EC* is shown twice in Fig. 3, but it is the same additional encoder EC*.

The additional encoder EC* therefore also extracts image features from the target representation TR.

A loss function LF is used to quantify (i) deviations between the synthetic representation SR and the target representation TR and (ii) deviations between the features extracted from the synthetic representation SR and the features extracted from the target representation TR.

By modifying parameters of the machine learning model MLM in an optimization procedure (e.g., a gradient descent procedure), the deviations can be reduced. The above steps are repeated for a plurality of input data and target data of a plurality of human livers until a stop criterion is met.

As already described, additional input data can be used to train the machine learning model MLM.

Such additional input data can comprise additional representations, such as one or more representations of the examination object which represent areas adjacent to the examination region represented by the representations R1 and/or R2.

Such other/additional input data can be information about the examination object, the examination region and/or the (measurement) method used to generate the input data and/or the target data.

Once the machine learning model has been trained, it can be used to predict, i.e., generate a synthetic representation of a new human liver. The term "new" means that no representation of the human liver was usually used when training the machine learning model.

Fig. 4 shows another exemplary and schematic embodiment of the computer-implemented method for generating a synthetic representation using a trained machine learning model.

The trained machine learning model MLM^{t} shown in Fig. 4 may have been trained in a method as described with respect to Fig. 3.

In a first step, a first representation R1ⁿ and a second representation R2ⁿ are received. It is possible that additional input data is received.

The first representation R1ⁿ may be a radiologic image, such as a CT image or an MRI image. The first representation R1ⁿ may represent a human liver at a first time point before or after application of a contrast agent.

The second representation R2ⁿ may also be a radiologic image, such as a CT image or an MRI image. The second representation R2ⁿ may represent the human liver at a second time point before or after application of the contrast agent.

The first representation R1ⁿ and the second representation R2ⁿ are inputted into the trained machine learning model MLM'.

The trained machine learning model MLM^{t} is configured and was trained to generate a synthetic representation SRⁿ based on the first representation R1ⁿ and the second representation R2ⁿ.

The synthetic representation SRⁿ may be a synthetic radiologic image, such as a synthetic CT image or a synthetic MRI image. The synthetic representation SRⁿ represents the human liver at a third time point before or after application of the contrast agent.

For example, the machine learning model MLM^{t} may be configured and trained to generate, based on a first representation R1ⁿ representing a human liver without contrast agent and a second representation R2ⁿ representing the liver at a time point after the application of a hepatobiliary contrast agent (e.g., in the arterial phase or portal venous phase or transitional phase), a synthetic representation SRⁿ of the human liver in the hepatobiliary phase after the application of the hepatobiliary contrast agent.

The synthetic representation SRⁿ can be output (e.g. displayed on a screen or printed using a printer) and/or stored in a data memory and/or transmitted to a separate computer system.

The procedures described herein may be performed in whole or in part using a computer system.

A "computer system" is an electronic data processing system that processes data by means of programmable calculation rules. Such a system typically comprises a "computer", which is the unit that includes a processor for carrying out logic operations, and peripherals.

In computer technology, "peripherals" refers to all devices that are connected to the computer and are used for control of the computer and/or as input and output devices. Examples thereof are monitor (screen), printer, scanner, mouse, keyboard, drives, camera, microphone, speakers, etc. Internal ports and expansion cards are also regarded as peripherals in computer technology.

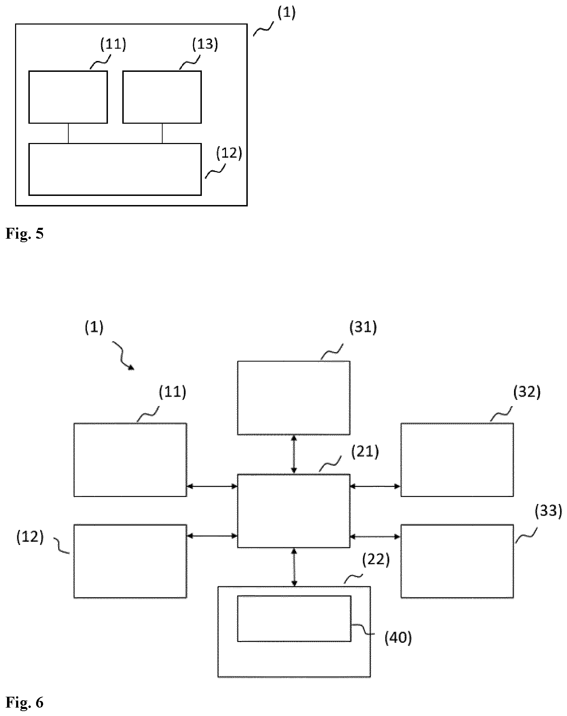

Fig. 5 shows by way of example and in schematic form a computer system according to the present disclosure.

The computer system (1) shown in Fig. 5 comprises a receiving unit (11), a control and calculation unit (12) and an output unit (13).

The control and calculation unit (12) serves for control of the computer system (1), coordination of the data flows between the units of the computer system (1), and for the performance of calculations.

The control and calculation unit (12) is configured:
- to provide a trained machine learning model, wherein the trained machine learning model is configured and trained to generate a synthetic image based on input data,
- to cause the receiving unit (11) to receive input data,
- to input the input data into the trained machine learning model,
- to receive a synthetic image as an output of the trained machine learning model,
- to cause the output unit (13) to output and/or store the synthetic image model and/or to transfer the synthetic image to a separate computer system.

Fig. 6 shows by way of example and in schematic form a further embodiment of the computer system. The computer system (1) comprises a processing unit (21) connected to a storage medium (22). The processing unit (21) and the storage medium (22) form a control and calculation unit, as shown in Fig. 5.

The processing unit (21) may comprise one or more processors alone or in combination with one or more storage media. The processing unit (21) may be customary computer hardware that is able to process information such as digital images, computer programs and/or other digital information. The processing unit (21) usually consists of an arrangement of electronic circuits, some of which can be designed as an integrated circuit or as a plurality of integrated circuits connected to one another (an integrated circuit is sometimes also referred to as a "chip"). The processing unit (21) may be configured to execute computer programs that can be stored in a working memory of the processing unit (21) or in the storage medium (22) of the same or of a different computer system.

The storage medium (22) may be customary computer hardware that is able to store information such as digital images (for example representations of the examination region), data, computer programs and/or other digital information either temporarily and/or permanently. The storage medium (22) may comprise a volatile and/or non-volatile storage medium and may be fixed in place or removable. Examples of suitable storage media are RAM (random access memory), ROM (read-only memory), a hard disk, a flash memory, an exchangeable computer floppy disk, an optical disc, a magnetic tape or a combination of the aforementioned. Optical discs can include compact discs with read-only memory (CD-ROM), compact discs with read/write function (CD-R/W), DVDs, Blu-ray discs and the like.

The processing unit (21) may be connected not just to the storage medium (22), but also to one or more interfaces (11, 12, 31, 32, 33) in order to display, transmit and/or receive information. The interfaces may comprise one or more communication interfaces (11, 32, 33) and/or one or more user interfaces (12, 31). The one or more communication interfaces may be configured to send and/or receive information, for example to and/or from an MRI scanner, a CT scanner, an ultrasound camera, other computer systems, networks, data storage media or the like. The one or more communication interfaces may be configured to transmit and/or receive information via physical (wired) and/or wireless communication connections. The one or more communication interfaces may comprise one or more interfaces for connection to a network, for example using technologies such as mobile telephone, wifi, satellite, cable, DSL, optical fibre and/or the like. In some examples, the one or more communication interfaces may comprise one or more close-range communication interfaces configured to connect devices having close-range communication technologies such as NFC, RFID, Bluetooth, Bluetooth LE, ZigBee, infrared (e.g. IrDA) or the like.

The user interfaces may include a display (31). A display (31) may be configured to display information to a user. Suitable examples thereof are a liquid crystal display (LCD), a light-emitting diode display (LED), a plasma display panel (PDP) or the like. The user input interface(s) (11, 12) may be wired or wireless and may be configured to receive information from a user in the computer system (1), for example for processing, storage and/or display. Suitable examples of user input interfaces are a microphone, an image- or video-recording device (for example a camera), a keyboard or a keypad, a j oystick, a touch-sensitive surface (separate from a touchscreen or integrated therein) or the like. In some examples, the user interfaces may contain an automatic identification and data capture technology (AIDC) for machine-readable information. This can include barcodes, radiofrequency identification (RFID), magnetic strips, optical character recognition (OCR), integrated circuit cards (ICC) and the like. The user interfaces may in addition comprise one or more interfaces for communication with peripherals such as printers and the like.

One or more computer programs (40) may be stored in the storage medium (22) and executed by the processing unit (21), which is thereby programmed to fulfil the functions described in this description. The retrieving, loading and execution of instructions of the computer program (40) may take place sequentially, such that an instruction is respectively retrieved, loaded and executed. However, the retrieving, loading and/or execution may also take place in parallel.

The computer system of the present disclosure may be designed as a laptop, notebook, netbook and/or tablet PC; it may also be a component of an MRI scanner, a CT scanner or an ultrasound diagnostic device.

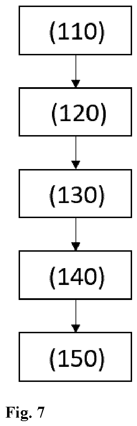

Fig. 7 shows schematically in the form of a flow chart a preferred embodiment of the computer-implemented method for generating a synthetic representation.

The method (100) comprises the steps:
(110) providing a trained machine learning model,
   ∘ wherein the trained machine learning model is configured and trained to generate a synthetic image based on input data,
   ∘ wherein the trained machine learning model was trained on training data, wherein the training data included, for each examination object of a plurality of examination objects, (i) input data and (ii) target data, wherein the target data included a target image of an examination region of the examination object,
   ∘ wherein training of the trained machine learning model for each examination object included the steps:
      ▪ determining target image features based in the target image,
      ▪ inputting the input data into the machine learning model,
      ▪ receiving a synthetic image as an output of the machine learning model,
      ▪ determining image features based on the synthetic image,
      ▪ reducing deviations (i) between the synthetic image and the target image and (ii) between the image features of the synthetic image and the target image features by modifying model parameters,
(120) receiving new input data,
(130) inputting the new input data into the trained machine learning model,
(140) receiving a new synthetic image as an output of the trained machine learning model,
(150) outputting and/or storing the new synthetic image and/or to transferring the new synthetic image to a separate computer system.

## Claims

1. A computer-implemented method, the method comprising:
- providing a machine learning model, wherein the machine learning model is configured to generate a synthetic image based on input data and parameters of the machine learning model,
- providing training data, wherein the training data comprises, for each examination object of a plurality of examination objects, (i) input data and (ii) target data, wherein the target data comprises a target image of an examination region of the examination object,
- for each target image: determining target image features based on the target image,
- training the machine learning model, wherein the training comprises, for each examination object of the plurality of examination objects:
∘ inputting the input data into the machine learning model,
∘ receiving a synthetic image as an output of the machine learning model,
∘ determining image features based on the synthetic image,
∘ reducing deviations (i) between the synthetic image and the target image and (ii) between the image features of the synthetic image and the target image features by modifying model parameters,
- outputting and/or storing the trained machine learning model and/or transferring the trained machine learning model to a separate computer and/or using the trained machine learning model to generate one or more synthetic medical images of one or more new examination objects.

2. The method of claim 1, further comprising:
- receiving new input data,
- inputting the new input data into the trained machine learning model,
- receiving a new synthetic image as output from the trained machine learning model,
- outputting and/or storing the new synthetic image and/or transmitting the new synthetic image to a separate computer system.

3. The method of any one of claims 1 or 2, wherein the image features of the synthetic image and the target image features comprise one or more of the following features: color histogram, greyscale histogram, texture features, edge features, SIFT features, HOG features, convolutional neural network features, low-level features, high-level features, content features, style features.

4. The method of any one of claims 1 to 3, wherein the image features of the synthetic image and the target image features are determined using a pre-trained convolutional neural network.

5. The method of any one of claims 1 to 4, wherein the image features of the synthetic image and the target image features are one or more feature maps generated by a pre-trained convolutional neural network.

6. The method of any one of claims 1 to 5, wherein each examination object is a living being, preferably a human, and the examination region is a part of the examination object.

7. The method of any one of claims 1 to 6, wherein the examination region is or comprises a liver, kidney, heart, lung, brain, stomach, bladder, prostate, intestine, thyroid, eye, breast or a part of said parts or another part of the body of a mammal, preferably a human.

8. The method of any one of claims 1 to 7, wherein the target image is a measured medical image and the synthetic image is a synthetic medical image.

9. The method of any one of claims 1 to 8, wherein the target image is a computer tomography image, an X-ray image, a magnetic resonance imaging image, a positron emission tomography image, a fluorescein angiography image, an optical coherence tomography image, a histological image, an ultrasound image, a fundus images or a microscopic image, and the synthetic image is a synthetic computer tomography image, a synthetic X-ray image, a synthetic magnetic resonance imaging image, a synthetic positron emission tomography image, a synthetic fluorescein angiography image, a synthetic optical coherence tomography image, a synthetic histological image, a synthetic ultrasound image, a synthetic fundus images or a synthetic microscopic image.

10. The method of any one of claims 1 to 9, wherein the input data comprises, for each examination object of the plurality of examination objects, at least one image of the examination region of the examination object.

11. The method of any one of claims 1 to 10, wherein the input data comprises, for each examination object of the plurality of examination objects, one or more radiologic images representing the examination region of the examination object before and/or after application of a first amount of a contrast agent, wherein the target image represents the examination region of the examination object after application of a second amount of a contrast agent, wherein the second amount differs from the first amount.

12. The method of any one of claims 1 to 11, wherein the input data comprises, for each examination object of the plurality of examination objects, a first radiologic image representing the examination region of the examination object without a contrast agent, a second radiologic image representing the examination region of the examination object after application of a first amount of a contrast agent, wherein the target image represents the examination region of the examination object after application of a second amount of a contrast agent, wherein the second amount differs from the first amount.

13. The method of any one of claims 1 to 12, wherein the input data comprises, for each examination object of the plurality of examination objects, one or more radiologic images representing the examination region of the examination object at one or more points in time before or after application of a contrast agent, wherein the target image represents the examination region of the examination object at another point in time before or after application of the contrast agent.

14. The method of any one of claims 1 to 13, wherein the input data comprises, for each examination object of the plurality of examination objects, a radiologic image representing the examination region of the examination object without contrast agent, one or more radiologic images representing the examination region of the examination object at one or more time points after application of a contrast agent, wherein the target image represents the examination region of the examination object at another time point after application of the contrast agent.

15. The method of any one of claims 1 to 14, wherein the input data comprises, for each examination object of the plurality of examination objects, one or more radiologic images representing the examination region of the examination object in a native phase, arterial phase, portal venous phase and/or transitional phase before and/or after application of a hepatobiliary contrast agent, wherein the target image represents the examination region of the examination object in a hepatobiliary phase after application of the hepatobiliary contrast agent.

16. The method of any one of claims 1 to 15, wherein the input data comprises, for each examination object of the plurality of examination objects, a radiologic image representing the examination region of the examination object in a radiologic examination using a first radiation dose, wherein the target image represents the examination region of the examination object in a radiologic examination using a second radiation dose, wherein the second radiation dose differs from the first radiation dose.

17. The method of any one of claims 1 to 16, wherein the input data comprises, for each examination object of the plurality of examination objects, a radiologic image of a first modality representing the examination region of the examination object, wherein the target image is a radiologic image of a second modality.

18. The method of any one of claims 1 to 17, comprising:
- providing a trained machine learning model,
∘ wherein the trained machine learning model is configured and trained to generate a synthetic image based on input data,
∘ wherein the trained machine learning model was trained on training data, wherein the training data included, for each examination object of a plurality of examination objects, (i) input data and (ii) target data, wherein the target data included a target image of an examination region of the examination object,
∘ wherein training of the trained machine learning model for each examination object included the steps:
▪ determining target image features based in the target image,
▪ inputting the input data into the machine learning model,
▪ receiving a synthetic image as an output of the machine learning model,
▪ determining image features based on the synthetic image,
▪ reducing deviations (i) between the synthetic image and the target image and (ii) between the image features of the synthetic image and the target image features by modifying model parameters,
- receiving new input data,
- inputting the new input data into the trained machine learning model,
- receiving a new synthetic image as an output of the trained machine learning model,
- outputting and/or storing the new synthetic image and/or to transferring the new synthetic image to a separate computer system.

19. A computer system comprising:
a processor; and
a storage medium that stores an application program configured to perform an operation when executed by the processor, said operation comprising the steps of:
- providing a machine learning model, wherein the machine learning model is configured to generate a synthetic image based on input data and parameters of the machine learning model,
- providing training data, wherein the training data comprises, for each examination object of a plurality of examination objects, (i) input data and (ii) target data, wherein the target data comprises a target image of an examination region of the examination object,
- for each target image: determining target image features based on the target image,
- training the machine learning model, wherein the training comprises, for each examination object of the plurality of examination objects:
∘ inputting the input data into the machine learning model,
∘ receiving a synthetic image as an output of the machine learning model,
∘ determining image features based on the synthetic image,
∘ reducing deviations (i) between the synthetic image and the target image and (ii) between the image features of the synthetic image and the target image features by modifying model parameters,
- outputting and/or storing the trained machine learning model and/or transferring the trained machine learning model to a separate computer and/or using the trained machine learning model to generate one or more synthetic medical images of one or more new examination objects.

20. A non-transitory computer readable storage medium having stored thereon software instructions that, when executed by a processor of a computer system, cause the computer system to execute the following steps:
- providing a machine learning model, wherein the machine learning model is configured to generate a synthetic image based on input data and parameters of the machine learning model,
- providing training data, wherein the training data comprises, for each examination object of a plurality of examination objects, (i) input data and (ii) target data, wherein the target data comprises a target image of an examination region of the examination object,
- for each target image: determining target image features based on the target image,
- training the machine learning model, wherein the training comprises, for each examination object of the plurality of examination objects:
∘ inputting the input data into the machine learning model,
∘ receiving a synthetic image as an output of the machine learning model,
∘ determining image features based on the synthetic image,
∘ reducing deviations (i) between the synthetic image and the target image and (ii) between the image features of the synthetic image and the target image features by modifying model parameters,
outputting and/or storing the trained machine learning model and/or transferring the trained machine learning model to a separate computer and/or using the trained machine learning model to generate one or more synthetic medical images of one or more new examination objects.
